Europäisches Patentamt

European Patent Office

Office européen des brevets

(11) Veröffentlichungsnummer: **0 338 105**
**A1**

## (12) EUROPÄISCHE PATENTANMELDUNG

(21) Anmeldenummer: **88106248.3**

(22) Anmeldetag: **20.04.88**

(51) Int. Cl.⁴: **H01R 13/62**

(43) Veröffentlichungstag der Anmeldung:
**25.10.89 Patentblatt 89/43**

(84) Benannte Vertragsstaaten:
**DE FR GB IT NL**

(71) Anmelder: **Erbe Elektromedizin GmbH.**
**Waldhörnlestrasse 17**
**D-7400 Tübingen(DE)**

(72) Erfinder: **Farin, Günter**
**Kapellenweg 15**
**D-7400 Tübingen(DE)**
Erfinder: **Fischer, Klaus**
**Immengasse 1**
**D-7270 Nagold-Emmingen(DE)**

(74) Vertreter: **Endlich, Fritz, Dipl.-Phys.**
**Postfach 1326 Blumenstrasse 8**
**D-8034 Germering(DE)**

(54) Steckverbindung.

(57) Es wird eine Steckverbindung mit einem Stecker (4) und einer Buchse (1) beschrieben, die insbesondere für elektromedizinische Geräte verwendbar ist. In der Steckverbindung ist mindestens ein Permanentmagnet (2) oder ein Elektromagnet (10) derart angeordnet, daß eine den Stecker (4), mit der Buchse (1) in der Kontaktlage haltende Magnetkraft ausgeübt werden kann.

Fig.1

## Steckverbindung

Die Erfindung betrifft eine Steckverbindung mit einem Stecker und einer Buchse, insbesondere für elektromedizinische Geräte.

Elektromedizinische Geräte enthalten im allgemeinen ein Grundgerät, beispielsweise ein Hochfrequenz-Chirurgiegerät, sowie Anwendungsteile wie Elektroden, elektrochirurgische Instrumente oder Operationsendoskope, welche mittels ein- oder mehradriger Kabel trennbar miteinander verbunden werden können. Elektrochirurgische Instrumente zur Anwendung der Hochfrequenz-Chirurgie sind beispielsweise monopolare oder bipolare Schneide- und/oder Koagulationselektroden, welche in Elektrodenhaltern bzw. Elektrodengriffen eingesetzt werden, oder monopolare und/oder bipolare Koagulationspinzetten und Koagulationsnadeln.

Zum Anschluß elektrochirurgischer Instrumente werden einadrige oder mehradrige flexible Kabel verwendet. In vielen Fällen ist das Kabel mittels Buchse und Stecker vom Instrument lösbar. Der Anschluß der Kabel an Hochfrequenz-Chirurgiegeräten erfolgt in der Regel ebenfalls mittels Buchse und Stecker.

Für monopolare Instrumente reichen einadrige Kabel, für bipolare Instrumente sind zweiadrige Kabel erforderlich. Soll ein Hochfrequenzgenerator eines Hochfrequenz-Chirurgiegeräts manuell vom Instrument aus mittels einer Taste am Instrument eingeschaltet werden, so ist auch für monopolare Instrumente mindestens ein zweiadriges Kabel erforderlich. Die Anzahl der Kontakt der jeweiligen Buchsen und/oder Stecker muß mindestens der Anzahl der Adern des Kabels entsprechen.

Zum Anschluß elektrochirurgischer Instrumente an Hochfrequenz-Chirurgiegeräten sind zahlreiche unterschiedlich konstruierte Buchse/Stecker-Systeme bekannt, welche man bezüglich ihrer Funktion in zwei Gruppen einteilen kann. Eine Gruppe bilden alle Buchse/Stecker-Systeme, bei welchen sowohl die elektrische Kontaktgabe als auch der mechanische Zusammenhalt von Buchse und Stecker durch federnde Kontaktelemente realisiert wird. Ein derartiges Buchse/Stecker-System ist beispielsweise in der DE-PS 18 03 292 beschrieben. Zu dieser ersten Gruppe gehören typischerweise Bananenstecker und entsprechnde Buchsen. Eine andere Gruppe bilden alle Buchse/Stecker-Systeme, bei welchen die Funktion der elektrischen Kontaktgabe unabhängig von der Funktion des mechanischen Zusammenhaltes von Buchse und Stecker realisiert wird, wobei der mechanische Zusammenhalt in der Regel als mechanische Verriegelung ausgeführt ist, beispielsweise als sogenannter Renkverschluß, Schraubverschluß, Bajonettverschluß oder Schnappverschluß. Zwischen den beiden Gruppen können Klinkenstecker und entsprechende Buchsen eingeordnet werden, welche beispielsweise zum Anschluß der neutralen Elektrode an Hochfrequenz-Chirurgiegeräten verwendet werden.

Insbesondere bei der Verwendung der bekannten Steckverbindungen für elektromedizinische Geräte der genannten Art können sich Schwierigkeiten ergeben. Die erste Gruppe von Steckverbindungen hat den Nachteil, daß die Kraft, mit welcher Buchse und Stecker zusammengesteckt bzw. voneinander getrennt werden müssen, infolge Toleranzen der Federkräfte der Kontaktfedern und/oder infolge von Verschleiß sehr weit variieren können. Insbesondere mehrpolige Steckverbindungen dieser Gruppe erfordern hohe Steck- bzw. Trennkräfte, was bei deren Anwendung im sterilen Operationsbereich insofern problematisch ist, als Chirurgen sowie deren Assistenten glatte, mechanisch leicht verletzbare Operationshandschuhe tragen, welche die Handhabung derartiger Steckverbindungen erschweren und unter Umständen zu Beschädigungen der Operationshandschuhe führen können.

Die zweite Gruppe von Steckverbindungen erfordert hohe Präzision der Mechanik, was relativ teuer ist. Außerdem sind diese Steckverbindungen pflegebedürftig und störanfällig, beispielsweise wenn Blut in die Mechanik gelangt und dort eintrocknet.

Bei allen bekannten Steckverbindungen wird ferner als nachteilig angesehen, daß Torsionen der Stecker in den Buchsen um die Steckrichtung herum relativ große Drehmomente erfordern oder praktisch unmöglich sind, so daß der Operateur das jeweilige Instrument nicht ohne mechanischen Widerstand beliebig um die Steckrichtung des jeweiligen Buchse/Stecker-Systems im die jeweils erforderliche Position drehen kann. Dies ist insbesondere dann hinderlich, wenn das Kabel den Stecker bzw. die Buchse am Instrument zur Rotationsachse abgewinkelt verläßt, weil dann das Gewicht des Kabels eine unangenehme Torisionskraft auf das Instrument verursachen kann, welche das Instrument in eine für den Chirurgen ungünstige Lage drehen kann.

Es ist deshalb Aufgabe der Erfindung, eine insbesondere bei elektromedizinischen Geräten unter möglichst weitgehender Vermeidung der genannten Nachteile und Schwierigkeiten verwendbare Steckverbindung zu schaffen, bei der zum Zusammenhalten in der Kontaktlage weder federnde Kontaktelemente noch eine aufwendige Mechanik erforderlich sind, und deren Elemente in Massenproduktion möglichst kostengünstig hergestellt werden können.

Diese Aufgabe wird erfindungsgemäß durch den Gegenstand des Patentanspruchs 1 gelöst. Vorteilhafte Weiterbildungen und zweckmäßige Ausgestaltungen der Erfindung sind Gegenstand der Unteransprüche.

Besondere Vorteile der Erfindung sind darin zu sehen, daß durch die Anordnung eines Elektromagneten oder eines Permanentmagneten in der Steckverbindung der Zusammenhalt in der kontaktlage durch Magnetkraft erfolgen kann, so daß keine mechanischen mittel zur Ausübung der erforderlichen Kontaktkraft benötigt werden. Ferner kann erreicht werden, daß eine relative Drehung zwischen Buchse und Stecker ohne das Erfordernis der Überwindung einer wesentlichen Reibungskraft erfolgen kann, was insbesondere die Handhabung eines elektrochirurgischen Instruments bei Durchführung einer Operation begünstigt. Bei der Verwendung von zwei Permanentmagneten kann ferner ein zusätzliche Sicherheitsfunktion erzielt werden, wenn beispielsweise an unterschiedlichen Instrumenten vorgesehene, im wesentlichen gleich ausgebildete Stecker eine Einsteckverbindung in eine Buchse ermöglichen sollen oder nicht. Die Erzielung einer Steckverbindung ist dann nicht möglich, wenn die beiden Kontaktflächen der Steckverbindung durch gleichnamige Magnetpole gebildet werden.

In vorteilhafter Weiterbildung der Erfindung kann ferner eine zusätzliche Arretierfunktion in der Kontaktlage erzielt werden, wenn ein mechanischer Anschlag zwischen einem Vorsprung an der Buchse und einem Rücksprung an dem Stecker derart vorgesehen werden, daß in der Kontaktlage ein Trennen von Buchse und Stecker nur dann möglich ist, wenn die Achse des Steckers in der Steckrichtung liegt. Dabei kann der Rücksprung auch in Form einer Längsnut in dem Stecker ausgebildet werden, in welcher ein entsprechend ausgebildeter Vorsprung an der Buchse beim Zusammenstecken geführt wird, so daß ein Trennen von Buchse und Stecker nur dann möglich ist, wenn eine vorherbestimmte relative Drehlage zwischen Buchse und Stecker vorhanden ist. Durch eine derartige, auch bei anderen bekannten Steckverbindungen verwendbare Arretierung kann erreicht werden, daß auch bei Ausübung einer normalerweise nicht auftretenden, verhältnismäßig großen Zugkraft eine Trennung der Steckverbindung mit praktisch ausreichender Sicherheit verhindert werden kann.

Folgende Funktionen bezüglich des mechanischen Zusammenhaltes von Buchse und Stecker können beispielsweise bei entsprechender Anordnung von Magneten in Buchse und/oder Stecker realisiert werden:

1. Ein Permanentmagnet pro Buchse/Stecker-System
Hiermit kann nur eine Funktion realisiert werden, und zwar eine magnetische Kraft, welche Buchse und Stecker zusammenzieht.

2. Je ein Permanentmagnet pro Buchse und Stecker
Hiermit können zwei verschiedene Funktionen realisiert werden

2.1 Die Permanentmagneten in der Buchse und im Stecker sind so gepolt, daß Buchse und Stecker einander zusammenziehen.

2.2 Die Permanentmagneten in der Buchse und im Stecker sind so gepolt, daß Buchse und Stecker einander abstoßen.

3. Ein Elektromagnet pro Buchse/Stecker-System
Hiermit können drei verschiedenen Funktionen realisiert werden

3.1 Ist der Elektromagnet eingeschaltet bzw. aktiviert, so werden Buchse und Stecker zusammengezogen.

3.2 Ist der Elektromagnet abgeschaltet bzw. nicht aktiviert, so werden Buchse und Stecker nicht zusammengezogen und können kraftlos getrennt werden.

3.3 Bei zusätzlicher Verwendung einer mechanischen Kraft, z.B. der Schwerkraft oder einer Federkraft, welche in entgegengesetzter Richtung der elektromagnetischen Kraft wirkt, wird Buchse und Stecker bei eingeschaltetem bzw. aktiviertem Elektromagneten zusammengezogen, wenn die elektromagnetische Kraft größer ist als die mechanische Kraft, und voneinander abgestoßen, wenn die mechanische Kraft größer ist als die elektromagnetische Kraft.

4. Je ein Permanentmagnet und ein Elektromagnet pro Buchse/Stekker-System
Hiermit können drei verschiedene Funktionen realisiert werden

4.1 Wird der Elektromagnet mittels Gleichstrom so aktiviert, daß seine Polarität der Polarität des Permanentmagneten gleichgerichtet ist, so ziehen Buchse und Stecker einander an

4.2 Wird der Elektromagnet mittels Gleichstrom so aktiviert daß seine Polarität der Polarität des Permanentmagneten entgegengerichtet ist, so stoßen Buchse und Stecker einander ab.

4.3 Ist in dem Teil eines Buchse/Stecker-System, im welchem ein Elektromagnet integriert ist, außerdem ein ferromagnetisches Teil enthalten, welches das Feld des Permanentmagneten des anderen Teiles des Buchse/Stecker-Systems im zusammengefügten Zustand erreicht, so kann die magnetische Kraft zwischen Permanentmagnet und ferromagnetischem Teil die Kraft für den Zusammenhalt von Buchse und Stecker bewirken und der Elektromagnet dazu genutzt werden, Buchse und Stecker mittels eines Stromimpulses in entsprechender Polung durch den Elektromagneten zu trennen.

Da der mechanische Zusammenhalt von Buchse und Stecker magnetisch erfolgt, können die elektrischen Kontakte des erfindungsgemäßen Buchse/Stecker-Systems so gestaltet und dimensioniert werden, daß deren Kontaktkraft einerseits ausreichend groß ist um einwandfreie elektrische Kontaktgabe zu gewährleisten, andererseits aber so klein sein darf, daß weder beim Zusammenstecken noch beim Trennen des jeweiligen Buchse/Stecker-Systems hinderliche Reibungskräfte überwunden werden müssen.

Die geringe Kontaktkraft und damit geringe Reibungskraft der kontakte ist auch insofern vorteilhaft, als hierdurch die Torsion zwischen Buchse und Stecker nicht gebremst wird, so daß Drehbewegungen des Instrumentes nicht durch Gegendrehmomente vom Kabel behindert werden.

Dieser Vorteil kann insbesondere bei einpoligen Buchse/Stekker-Systemen, bei welche möglichst reibungsfreie Torsionen zwischen Buchse und Stecker um die Steckrichtung herum gefordert wird, genutzt werden, wenn die Richtung der Kontaktschliessung in die Richtung der magnetischen Anziehungskraft. gelegt wird. Die Kontaktschließungskraft ist dann identisch der Kraft, welche Buchse und Stecker zusammenzieht.

Um zu verhindern, daß erfindungsgemäße Buchse/Stecker-Systeme unbeabsichtigt z.B. durch ruckartigen Zug am Kabel, getrennt werden, kann dieses System durch einen mechanischen Anschlag ausgestattet werden, welcher wie ein Widerhaken wirkt und das Trennen von Buchse und Stecker nicht möglich ist, wenn die Trennkraft in einem von der Steckrichtung des Buchse/Stecker-System abweichenden Raumwinkel wirkt, wie es bei unbeabsichtigtem Zug am Kabel in der Regel der Fall ist.

Die Erfindung wird in Verbindung mit Zeichnungen an Ausführungsbeispielen näher erläutert. Da Konstruktionselemente von Buchsen und Steckern sowie die damit ausgestatteten elektromedizinischen Instrumente, Kabel und Geräte allgemein bekannt sind, werden im folgenden insbesondere die erfindungsrelevanten Details des erfindungsgemäßen Buchse/Stecker-Systems dargestellt und beschrieben. Es Zeigen :

Fig.1 eine schematische Darstellung eines Ausführungsbeispieles des erfindungsgemäßen Buchse/Stecker-Systems mit einem Permanentmagneten in der Buchse und einem ferromagnetischen Teil im Stecker.

Fig.2 eine schematische Darstellung eines Ausführungsbeispieles des erfindungsgemäßen Buchse/Stecker-Systems mit je einem Permanentmagneten in Buchse und Stecker, wobei die beiden Permanentmagneten so gepolt sind, daß Buchse und Stecker einander anziehen.

Fig.3 eine schematische Darstellung des erfindungsgemäßen Buchse/Stecker-Systems mit je einem Permanentmagneten in Buchse und Stecker, wobei die beiden Permanentmagneten so gepolt sind, daß Buchse und Stecker einander abstoßen.

Fig.4 eine schematische Darstellung des erfindungsgemäßen Buchse/Stecker-Systems mit einem Elektromagneten in der Buchse und einem ferromagnetischen Teil im Stecker.

Fig.5 eine schematische Darstellung des erfindungsgemäßen Buchse/Stecker-Systems mit einem Elektromagneten ohne ferromagnetischem Teil in der Buchse und einem Permanentmagneten im Stecker, wobei der Elektromagnet beispielsweise so gepolt ist, daß Buchse und Stecker einander anziehen, solange der Elektromagnet aktiviert ist.

Fig.6 eine schematische Darstellung des erfindungsgemäßen Buchse/Stecker-Systems mit einem Elektromagneten ohne ferromagnetischem Teil in der Buchse und einem Permanentmagneten im Stecker, wobei der Elektromagnet beispielsweise so gepolt ist, daß Buchse und Stecker einander abstoßen, solange der Elektromagnet aktiviert ist.

Fig.7 eine schematische Darstellung des erfindungsgemäßen Buchse/Stecker-Systems mit einem Elektromagneten und einem ferro magnetischen Teil in der Buchse und einem Permanentmagneten im Stecker.

Fig.8 eine schematische Darstellung des erfindungsgemäßen Buchse/Stecker-Systems mit einem Elektromagneten in der Buchse und einem ferromagnetischen Teil im Stecker, wobei zusätzlich eine mechanische Feder so angeordnet ist, daß Buchse und Stecker bei nicht aktiviertem Elektromagneten durch die mechanische Feder getrennt werden.

Fig.9 eine schematische Darstellung eines Ausführungsbeispiels eines zweipoligen Buchse/Stecker-Systems entsprechend der Erfindung.

Fig.10 bis 13 eine schematische Darstellung einer Ausgestaltung des erfindungsgemäßen Buchse/Stecker-Systems zur Verhinderung unbeabsichtigter Trennung von Buchse und Stecker.

In allen Zeichnungen sind gleichwirkende Teile mit gleichen Bezugsziffern gekennzeichnet.

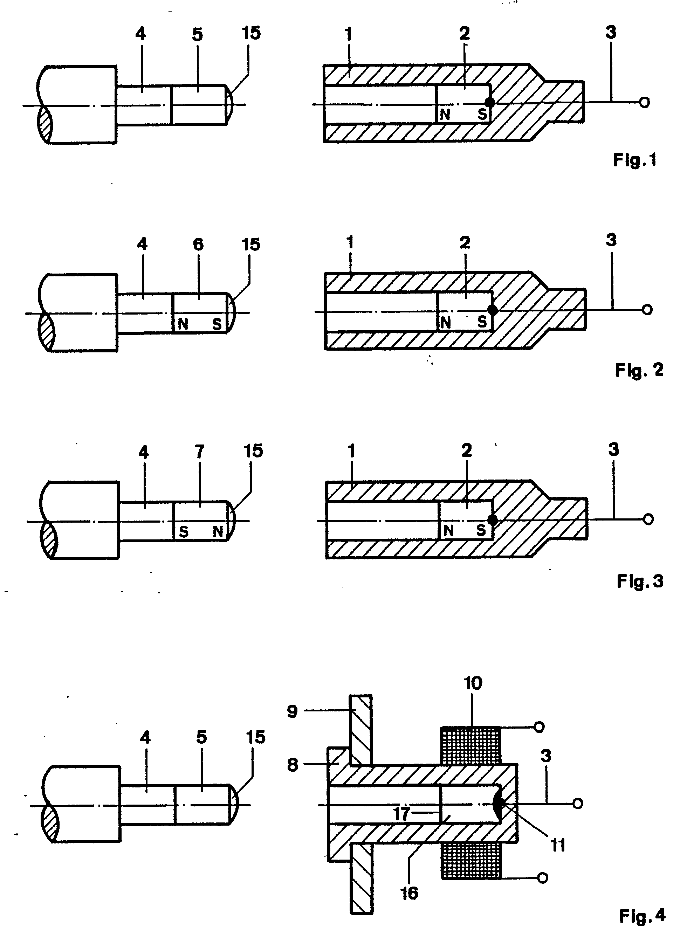

In Figur 1 ist schematisch ein Ausführungsbeispiel eines einpoligen Buchse/Stecker-Systems, wie es beispielsweise an einpoligen Elektrodengriffen, monopolaren Koagulationspinzetten oder an Resektoskopen verwendbar ist, dargestellt. Innerhalb des Buchsengehäuses 1 ist ein Permanentmagnet 2 angeordnet, welcher auch als elektrischer Kontakt innerhalb der Buchse 1 benutzt wird. Hierzu ist er elektrisch an das Kabel 3 angeschlossen. Der Stecker 4 besteht zumindest in seinem vorderen Bereich 5 aus ferromagnetischem Metall. Der elektrische Kontakt entsteht an der konvexen Stirnfläche 15 des Steckers, wenn diese den Permanentmagneten 2 berührt. Mit Rücksicht auf gute Korrosionsbeständigkeit und geringem elektrischem Übergangswiderstand ist es empfehlenswert, insbesondere die Kontaktflächen von Buchse und Stecker mit geeigneten Metallen zu galvanisieren.

In diesem Ausführungsbeispiel kann die Polarität des Permanentmagneten bei gleicher Wirkung getauscht werden. Außerdem ist es für die Wirkung unerheblich, ob der Permanentmagnet 2, wie in Figur 1 dargestellt, in der Buchse 2 oder im Stecker 4 anstelle des ferromagnetischen Teiles 5 angeordnet ist, wobei dann das ferromagnetische Teil in dert Buchse 1 anstelle des Permanentmagneten angeordnet sein muß.

Ist der Querschnitt des Steckers wie auch der Buchse rund, so können Buchse und Stecker mit geringer Reibung zwischen Buchse und Stecker um die Steckrichtung herum gegeneinander verdreht werden.

Die in Figur 2 und 3 schematisch dargestellten Ausführungsbeispiele unterschieden sich vom Ausführungsbeispiel in Figur 1 lediglich dadurch, daß nicht nur in der Buchse oder nur im Stecker ein Permanentmagnet angeordnet ist, sondern sowohl in der Buchse als auch im Stecker. Sind beide Permanentmagneten 2 und 6, wie in Figur 2 dargestellt, in gleicher Richtung gepolt, so ziehen sich Buchse und Stecker einander an. Sind beide Permanentmagneten 2 und 7, wie in Figur 3 dargestellt, in entgegengesetzter Richtung gepolt, so stoßen Buchse und Stecker einander ab. Diese Wirkung kann dazu benutzt werden, erfindungsgemäße Buchse/Stecker-Systeme mit im wesentlichen gleichen Bauelementen so zu gestelten, daß bestimmungsgemäße Verbindungen möglich sind und nicht bestimmungsgemäße Verbindungen nicht möglich sind. Beispielsweise kann ein Instrument, welches bestimmungsgemäß an die in Figur 2 oder Figur 3 dargestellte Buchse 1 angeschlossen werden darf mit der in Figur 2 dargestellten Polung des Permanentmagneten 6 des Steckers ausgestattet sein. Dagegen muß ein Instrument, das nicht an der gleichen Buchse betrieben werden darf, mit einem Stecker ausgestattet sein, dessen Magnet 7, wie in Figur 3 dargestellt, dem Magneten 2 in der Buchse entgegengepolt ist.

In Figur 4 ist schematisch ein weiteres Ausführungsbeispiel des erfindungsgemäßen Buchse/Stecker-Systems dargestellt. Der Stecker 4 entspricht dem in Figur 1 dargestellten und beschriebenen Stecker. Die Buchse 8 ist in diesem Ausführungsbeispiel eine Geräte-Einbaubuchse, welche besipielsweise in der Frontplatte 9 eines Gerätes installiert sein kann. Die magnetische Kraft wird bei dieser Buchse mittels eines Elektromagneten 10 erzeugt. Die elektrische Kontaktgabe erfolgt beispielsweise an einem zentral in der Buchse 8 angeordneten Kontakt 11 oder, falls der Elektromagnet einen ferromagnetischen Kern 16 enthält, an der Kontaktfläche 15 des Steckers 4 zugewandten Kontaktfläche 17.

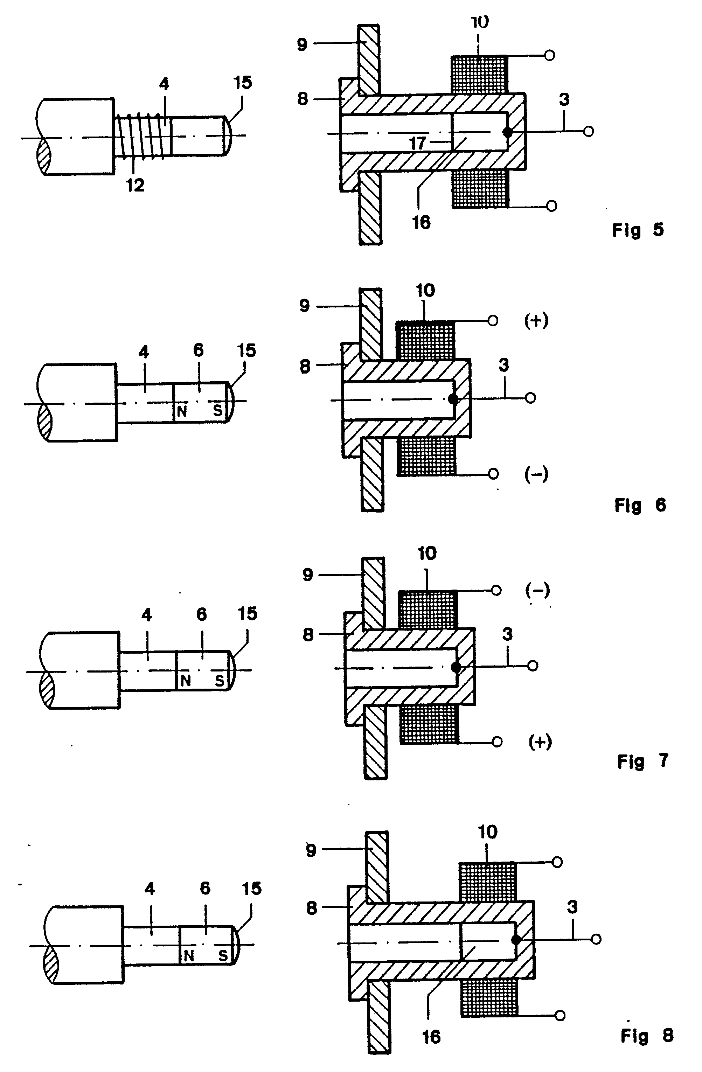

Ist die Buchse 8 oder der Stecker 4 zusätzlich mit einer mechanischen Federkraft 12 ausgestattet, wie in Figur 5 dargestellt, welche entgegen der Magnetkraft wirkt, so kann der elektrische Kontakt automatisch getrennt werden, indem der Elektromagnet 10 abgeschaltet wird.

Ist der Stecker 4, wie in Figur 6 schematisch dargestellt, mit einem Permanentmagneten 6 ausgestattet, so kann je nach Polarität des Elektromagneten 10 der Stecker 4 mit dem Permanentmagneten 6 in die Buchse 8 hineingezogen werden, wie in Figur 6 dargestellt, oder, wie in Figur 7 dargestellt, aus der Buchse 8 herausgeschoben werden.

Das in Figur 8 schematisch dargestellte Ausführungsbeispiel des erfindungsgemäßen Buchse/Stecker-Systems, bei welchem der Stecker 4 mit einem Permanentmagneten 6 und die Buchse 8 mit einem Elektromagneten 10 ausgestattet sind, wobei der Elektromagnet einen ferromagnetischen Kern 16 enthält, zieht der Permanentmagnet 6 den Stecker bereits in die Buchse, wenn der Elektromagnet abgeschaltet ist. Je nach Polung des Elektromagneten 10 wird die Anziehungskraft zwischen Buchse und Stecker vergrößert oder verkleinert. Mittels eines kräftigen Stromstoßes durch den Elektromagneten in geeigneter Polung kann der Stecker automatisch aus der Buchse ausgeworfen werden.

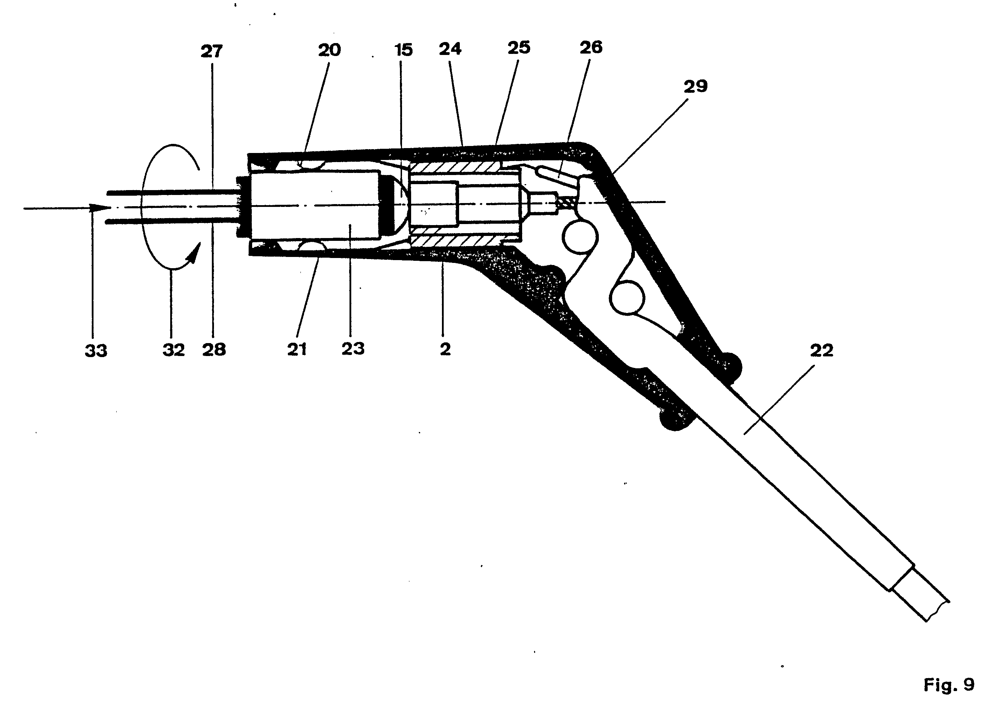

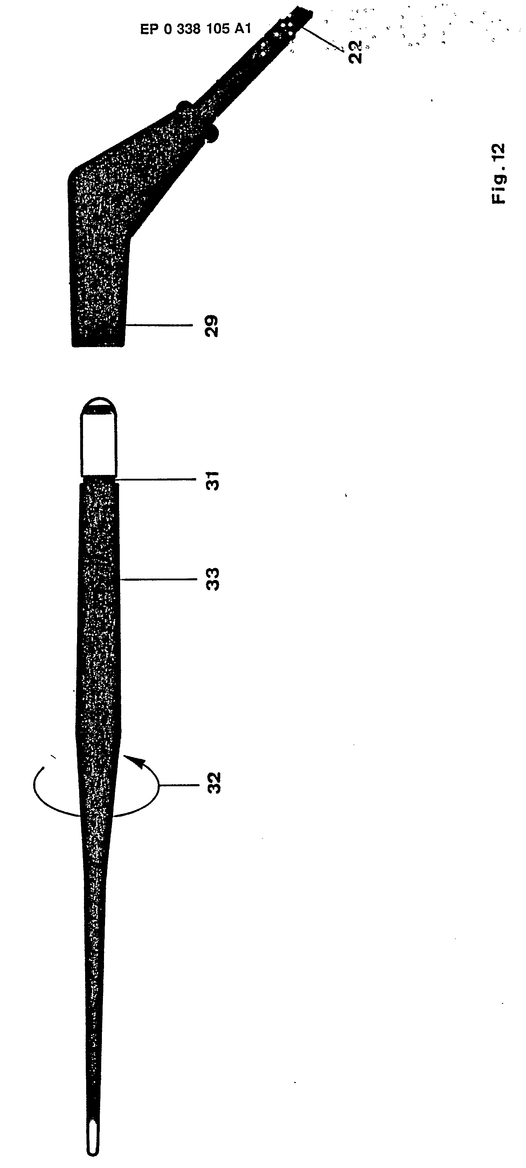

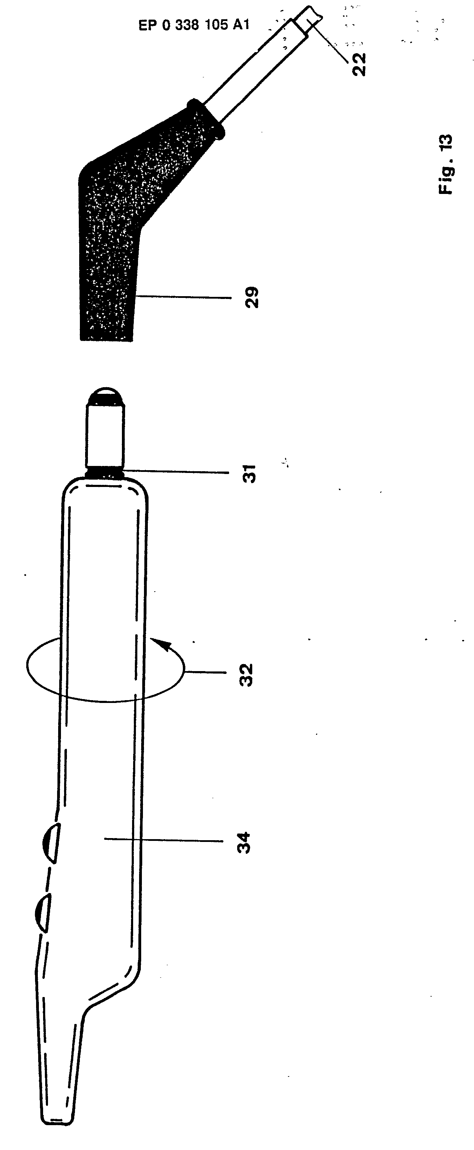

In Figur 9 ist schematisch ein Ausführungsbeispiel eines zweipoligen Buchse/Stecker-Systems dargestellt, wie es beispielsweise bei Elektrodengriffen mit Tasten zum Aktivieren von Hochfrequenzgeneratoren in Hochfrequenz-Chirurgiegeräten oder bei bipolaren Koagulationsinstrumenten verwendet werden kann. Zusätzlich zu dem Kontakt in dem oben beschriebenen einpoligen Buchse/Stecker-System, bei welchem dieser einzige Kontakt zentral in Steckrichtung angeordnet ist, ist mindestens ein zweiter elektrischer Kontakt 20 angeordnet, welcher an die zweite Ader 26 des zweiadrigen Kabels 22 elektrisch leitfähig angeschlossen ist. Bei einem runden Buchse/Stecker-System, bei welchem eine Verdrehung 32 zwischen Buchse und Stecker um die Steckrichtung 33 herum bei Einwirkung von Torsionskräften mit möglichst geringer Reibungs- bzw. Bremskraft möglich sein soll, kann der zusätzliche Kontakt 20 beispielsweise auf einer runden Kontakthülse 23 des Steckers gleiten. Während die Kontaktgabe zwischen dem gleichzeitig als Kontakt wirkenden Permanentmagneten 2 und dem zentralen Kontakt 15 des Steckers infolge der Magnetkraft weitgehend prellfrei ist, ist es vorteilhaft, die Kontaktgabe des Kontaktes 20 zur Kontakthülse 23 des Steckers durch zwei oder mehr Kontaktfedern 20, 21, welche gleichmäßig um den Umfang der Kontakthülse 23 angeordnet sind, zu realisieren, so daß auch hier möglichst prellfreie Kontaktgabe gewährleistet ist. Die Kontaktfedern 20, 21, und eventuell weitere, gleichwirkende Kontaktfedern, sind durch einen gemeinsamen Kontaktring 25 in der Buchse 29 an die zweite Ader 26 des Kabels 22 angeschlossen. Die beiden Kontakte 15 und 23 des Steckers sind durch die Leitungen 27 und 28 oder direkt an die relevanten elektromedizinischen Anwendungsteile angeschlossen, beispielsweise an bipolare Koagulationspinzetten, wie in Figur 12 dargestellt, oder an Elektrodengriffen, wie in Figur 13 dargestellt.

Mit mehreren voneinander isolierten Kontaktringen am Stecker und entsprechend vielen voneinander isolierten Kontaktfedern in der Buchse können auch mehrpoligere Buchse/Stecker-Systeme entsprechend der Erfindung realisiert werden.

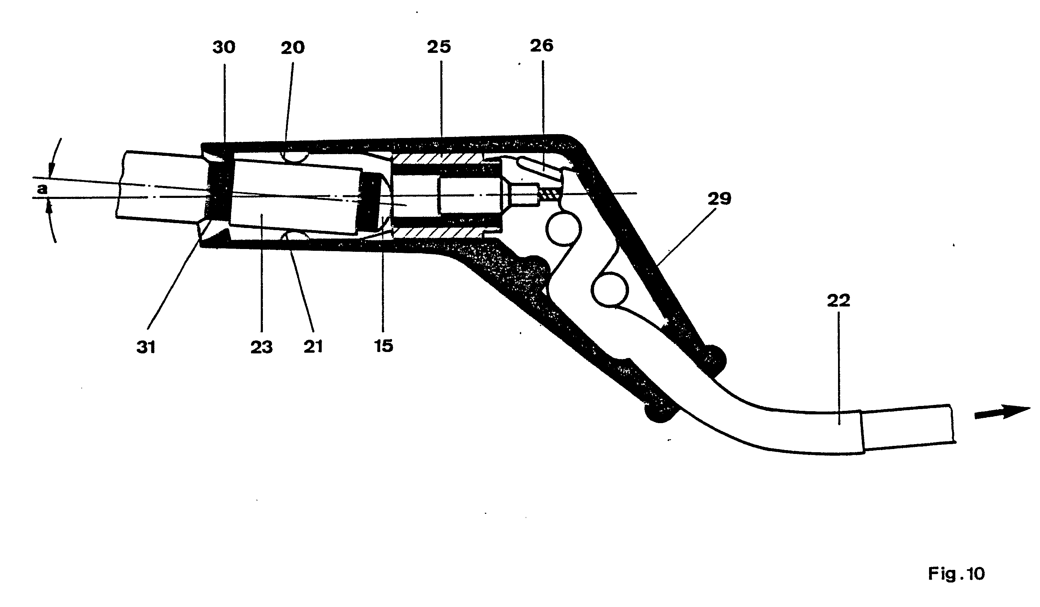

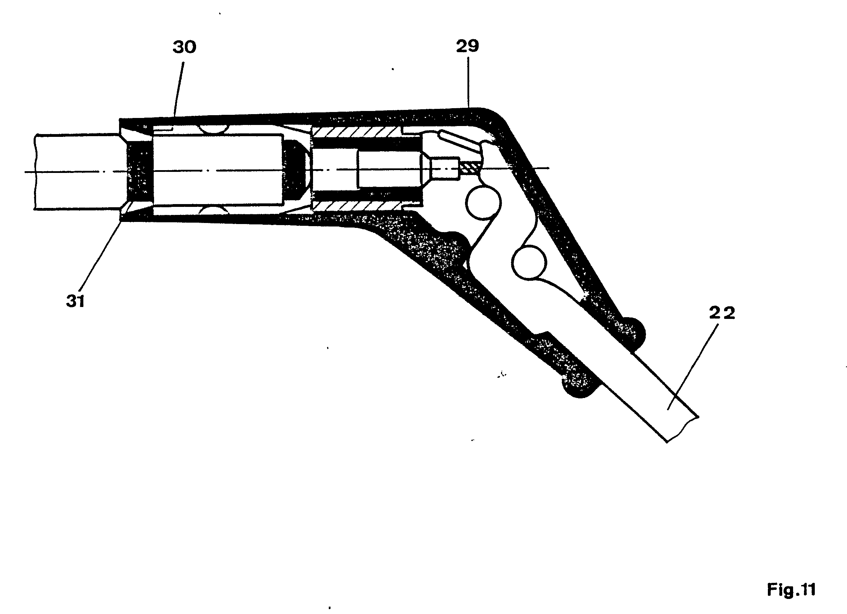

Ist die Verdrehung zwischen Buchse und Stecker um die Steckrichtung herum nicht relevant oder soll diese gar verhindert werden, so sind auch nicht runde Buchsen und Stecker entsprechend der Erfindung realisierbar. Die Figuren 10 bis 13 zeigen schematisch dargestellt eine weitere Ausgestaltung des erfindungsgemäßen Buchse/Stecker-Systems. Um zu verhindern, daß bei unbeabsichtigtem Zug am Kabel 22 oder am elektromedizinischen Instrument, beispielsweise an einer bipolaren Koagulationspinzette 33 oder an einem Elektrodengriff 34, Buchse und Stecker getrennt werden, ist der Stecker proximal zum Instrument mit einer Nut 31 und die Buchse mit einem widerhakenähnlichen Vorsprung 30 ausgestattet. Die Nut 31 ist vorzugsweise um den gesamten Umfang des Steckers angelegt. Der Vorsprung 30 in der Buchse ist vorzugsweise um den gesamten inneren Umfang der Buchsenöffnung herum angelegt. Auf diese Weise können Buchse und Stecker nicht ohne Gewalt getrennt werden, wenn, wie in Figur 10 dargestellt, Buchse und Stecker durch einen Zug am Kabel 22 gegeneinander um einen Winkel a verkantet werden, dagegen leicht getrennt werden, wenn, wie in Figur 11 dargestellt, Buchse und Stecker bestimmungsgemäß ohne Verkantung auseinandergezogen werden.

In den Figuren 12 und 13 sind Ausführungsbeispiele von Buchse und Stecker inclusive elektromedizinischer Instrumente, wie beispielsweise einer bipolaren Koagulationspinzette 33 oder einem Elektrodengriff 34 mit Tasten, dargestellt. In diesen beiden Fällen sind die Stecker sowie die Buchsen rund ausgeführt, so daß das Instrument 33 bzw. 34 und die Buchsen gegeneinander verdrehbar sind. Das erfindungsgemäße Buchse/Stecker-System kann jedoch auch mit nicht rundem Buchsen- und Steckerquerschnitt ausgeführt werden.

## Ansprüche

1. Steckverbindung mit einem Stecker und einer Buchse, insbesondere für elektromedizinische Geräte, **dadurch gekennzeichnet,** daß in der Steckverbindung mindestens ein Permanentmagnet (2;6) oder ein Elektromagnet (10) derart angeordnet ist, daß eine den Stecker (4) mit der Buchse (1;8) in der Kontaktlage haltende Magnetkraft ausübbar ist.

2. Steckverbindung nach Anspruch 1, **dadurch gekennzeichnet,** daß der Permanentmagnet (2) in Steckrichtung gesehen am Ende der Einschuböffnung der Buchse angeordnet ist, und daß das einzuschiebende Ende des Steckers durch ein ferromagnetisches Teil (5) oder einen zweiten Permanentmagnet (6) gebildet ist.

3. Steckverbindung nach Anspruch 1, **dadurch gekennzeichnet,** daß der Elektromagnet (10) an der Buchse (8) angeordnet ist, und daß das einzuschiebende Ende des Steckers durch ein ferromagnetisches Teil (5) oder einen Permanentmagnet (6) gebildet ist.

4. Steckverbindung nach Anspruch 3, **dadurch gekennzeichnet,** daß der Stecker in der Kontaktlage durch eine Feder (12) in einer der Richtung der elektromagnetischen Kraft entgegengesetzten Richtung vorgespannt ist.

5. Steckverbindung nach Anspruch 3 oder 4, **dadurch gekennzeichnet,** daß am Ende der Einschuböffnung der Buchse ein eine Kontaktfläche (17) bildender ferromagnetischer Kern (16) angeordnet ist.

6. Steckverbindung nach einem der vorhergehenden Ansprüche, **dadurch gekennzeichnet,** daß eine konvexe Kontaktfläche (15) an der Stirn des Steckers in Steckrichtung gesehen vorgesehen ist, und daß eine konvexe oder plane Kontaktfläche (17) an dem Permanentmagnet (2) bzw. dem ferromagnetischen Kern (16) in der Buchse vorgesehen ist.

7. Steckverbindung nach einem der vorhergehenden Ansprüche, **dadurch gekennzeichnet,** daß für eine mehrpolige Steckverbindung zwei oder mehr gleichmäßig entlang dem Innenumfang der Buchse angeordnete Kontaktfedern (20,21) vorgesehen sind, die in der Kontaktlage mit einer betreffenden Kontakthülse (23) an dem Stecker in Berührung stehen.

8. Steckverbindung nach einem der vorhergehenden Ansprüche, **dadurch gekennzeichnet,** daß der Stecker und die Einschuböffnung der Buchse kreiszylindrisch und derart ausgebildet sind, daß Stecker und Buchse mit geringer Reibung um die Steckrichtung als Achse gegeneinander verdrehbar sind.

9. Steckverbindung nach einem der vorhergehenden Ansprüche, **dadurch gekennzeichnet,** daß ein mechanischer Anschlag zwischen einem Vorsprung (30) an der Buchse und einem Rücksprung (31) an dem Stecker derart vorgesehen sind, daß in der Kontaktlage ein Trennen von Buchse und Stecker nur dann möglich ist, wenn die Achse des Steckers in der Steckrichtung liegt oder eine vorherbestimmte relative Drehlage zwischen Buchse und Stecker vorhanden ist.

10. Steckverbindung nach einem der vorhergehenden Ansprüche, **dadurch gekennzeichnet,** daß der Stecker eine das Ende einer bipolaren Pinzelle (33) oder eines Elektrodengriffs (34) umgebende und daran befestigte Kontakthülse (23) aufweist.

11. Steckverbindung nach einem der Ansprüche 1 bis 9, **dadurch gekennzeichnet,** daß die Buchse (8) an einer Frontplatte (9) eines Hochfrequenz-Chirurgiegeräts befestigt ist.

12. Steckverbindung nach einem der vorhergehenden Ansprüche, **dadurch gekennzeichnet,** daß Stecker und/oder Buchsen mit derart relativ unterschiedlichen Polungen der Magnetpole vorgesehen sind, daß die Kontaktlage nur dann bewirkt werden kann, wenn die betreffenden beiden Kontaktflächen (15,17) ungleichnamig gepolt sind.

Fig.1

Fig. 2

Fig.3

Fig.4

EP 0 338 105 A1

Fig 5

Fig 6

Fig 7

Fig 8

Fig. 9

30  20        25    26

a

29

31  23  21  15

22

EP 0 338 105 A1

Fig. 10

**30**

**29**

**31**

**22**

EP 0 338 105 A1

**Fig.11**

Fig.12

**34**

**32**

**31**

**29**

**22**

EP 0 338 105 A1

**Fig. 13**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-3 622 948  (EICHHOLZ)<br>* Figuren 1,2; Spalte 3, Zeile 27 - Spalte 4, Zeile 9 *<br>--- | 1-3,6, 12 | H 01 R   13/62 |
| X | US-A-4 653 503  (HEATH)<br>* Figuren 1,2,5; Spalte 4, Zeile 63 - Spalte 7, Zeile 4 *<br>--- | 1-3,5,8 | |
| A | DE-A-1 764 374  (LINN)<br>* Ganzes Dokument *<br>----- | 1-4,6,8 ,9 | |

RECHERCHIERTE SACHGEBIETE (Int. Cl.4)

. H 01 R   13/00

Der vorliegende Recherchenbericht wurde für alle Patentansprüche erstellt

| Recherchenort | Abschlußdatum der Recherche | Prüfer |
|---|---|---|
| BERLIN | 16-12-1988 | HAHN G |